# EUROPEAN PATENT APPLICATION

(11) **EP 3 998 088 A1**
(43) Date of publication of application: **18.05.2022**
(21) Application number: 20844573.4
(22) Date of filing: 01.07.2020
(51) Int. Cl.: A61L 9/20

(54) **GAS TREATING APPARATUS AND GAS TREATING METHOD**

(30) Priority: 22.07.2019 JP 2019134527
(71) Applicant: Ushio Denki Kabushiki Kaisha, Tokyo 100-8150 (JP)
(72) Inventor: NAITO,Keisuke, Tokyo 100-8150 (JP)
(74) Representative: Tomerius, Isabel
(86) International application number: PCT/JP2020/025873
(87) International publication number: WO 2021/014912

(57) **Abstract**

Provided is a gas treating apparatus that, in order to decompose a to-be-treated gas containing VOC by using light having a main peak wavelength of 160 to 180 nm, supplies moisture in the supply mode and supply quantity. A gas treating apparatus (10, 20, 30) includes: a gas mixing space (3) where a first gas containing a VOC and oxygen and a second gas containing water vapor are mixed to generate a gas to be treated that contains a VOC, oxygen, and 9 g/m³ or more of water vapor; a light source radiating light having a main peak wavelength of 160 to 180 nm; and a gas treatment space (7) where the gas to be treated is guided to a region within 10 mm of a light-emitting surface of the light source and the gas to be treated guided to the region is irradiated with the light.

## Description

### TECHNICAL FIELD

This invention relates to a gas treating apparatus and a gas treating method, and more particularly to an apparatus and method for treating VOC-containing gases to be treated.

### BACKGROUND ART

Gas treating apparatuses that decompose gases containing chemical pollutants such as malodorous components by ultraviolet have been developed in recent years. It has hitherto been known, in such a gas treating apparatus, to supply moisture for enhancing the gas treatment efficiency. For example, Patent Document 1 listed below describes an apparatus in which a kitchen waste gas is passed through a cleaning container sprayed with a cleaner solution containing ozone gas and titanium dioxide and irradiated with ultraviolet to remove the odor. Patent Document 2 listed below describes efficient decomposition and removal of chemical pollutants by ultraviolet irradiation, in which cleaning water is supplied to an ultraviolet irradiation space to facilitate generation of hydroxyl radicals.

### PRIOR ART DOCUMENTS

### PATENT DOCUMENT

Patent Document 1: JP-A-2002-143637
Patent Document 2: JP-A-2006-204683

### SUMMARY OF THE INVENTION

### PROBLEMS TO BE SOLVED BY THE INVENTION

The present inventor is considering a use of light having a main peak wavelength of 160 to 180 nm, which belongs to the wavelength range of vacuum ultraviolet and which is different from that of the Patent Documents mentioned above, in order to decompose gases to be treated which contain VOCs that are chemical pollutants.

There are currently no findings regarding efficient treatment of VOC-containing gases to be treated with the use of light having a main peak wavelength of 160 to 180 nm.

An object of the present invention is to provide a gas treating apparatus and a gas treating method whereby VOC-containing gases to be treated can be treated efficiently with the use of light having a main peak wavelength of 160 to 180 nm.

### MEANS FOR SOLVING THE PROBLEMS

A gas treating apparatus according to the present invention includes: a gas mixing space where a first gas containing a VOC and oxygen and a second gas containing water vapor are mixed to generate a gas to be treated that contains a VOC, oxygen, and 9 g/m³ or more of water vapor;
a light source radiating light having a main peak wavelength of 160 to 180 nm; and
a gas treatment space where the gas to be treated is guided to a region within 10 mm of a light-emitting surface of the light source and the gas to be treated guided to the region is irradiated with the light.

In the case where the gas to be treated is the first gas containing a VOC and oxygen (e.g., air containing a VOC mixed therein), if the gas to be treated contains moisture, irradiation of the gas to be treated with the light having a main peak wavelength of 160 to 180 nm produces a large amount of hydroxyl radicals. This reaction will be discussed later with reference to Formula (1) to Formula (5) in the section "EMBODYIMENTS FOR CARRYING OUT INVENTION."

The large amount of hydroxyl radicals thus produced is considered to provide a high efficiency of decomposing VOCs contained in the gas to be treated.

When imparting moisture to the gas to be treated, it is preferable to do so under conditions that are particularly suited to increase the efficiency of decomposing VOCs. Through intensive research, the present inventor found out that irradiation of a gas to be treated with light having a main peak wavelength of 160 to 180 nm, in a condition where the gas to be treated includes a second gas containing 9 g/m³ or more of water vapor, distinctly improved the efficiency of decomposing VOCs, and thus arrived at the present invention.

The gas treating apparatus described above supplies moisture in vapor (gaseous) form, so that the probability of contact between light and water molecules is higher and hydroxyl radicals are more readily generated. Since the gas to be treated contains water vapor of 9 g/m³ or more, a large amount of VOCs can be removed.

The gas treating apparatus may further include a second gas supply unit that supplies the second gas to the gas mixing space,
a water vapor amount detector that detects a water vapor amount contained in the first gas, and
a controller that adjusts an amount of water vapor contained in the second gas supplied from the second gas supply unit based on a detection result of the water vapor amount detector.

With the gas treating apparatus described above, a necessary water vapor amount for efficient removal of VOCs can be added to the second gas in accordance with the water vapor amount contained in the first gas. This can also prevent condensation of the gas to be treated that can happen in the event of an excess of supply of water vapor, which reduces the likelihood of damaging the light source.

A gas treating method according to the present invention includes:
an introducing step (a) of causing a gas to be treated that contains a VOC, oxygen, and 9 g/m³ or more of water vapor to flow into a region within 10 mm of a light-emitting surface of a light source radiating light having a main peak wavelength of 160 to 180 nm; and
an irradiation step (b) of irradiating the gas to be treated that has flowed into the region with the light.

With the gas treating method described above, a large amount of hydroxyl radicals are generated, so that a high efficiency of decomposing VOCs contained in the gas to be treated can be achieved. Thus, a large amount of VOCs can be removed.

The gas treating method described above may further include a gas to be treated generation step (c), in which a first gas containing a VOC and oxygen and a second gas containing water vapor are mixed to produce the gas to be treated, before the irradiation step (b).

Further, the gas to be treated generation step (c) may include:
a detection step of detecting a water vapor amount contained in the first gas; and
a step of adjusting a water vapor amount in which a water vapor amount contained in the second gas is adjusted based on the water vapor amount detected in the detection step.

With the gas treating method described above, a necessary water vapor amount for efficient removal of VOCs can be added in accordance with the water vapor amount contained in the first gas. This can also prevent condensation of the gas to be treated that can happen in the event of an excess of supply of water vapor, which reduces the likelihood of damaging the light source.

The water vapor amount contained in the gas to be treated that is guided to a region within 10 mm of the light-emitting surface should preferably be 20 g/m³ or less. This makes it easier to prevent condensation of the gas to be treated, and reduces the likelihood of damaging the light source.

The VOCs may include toluene or formaldehyde.

### EFFECT OF THE INVENTION

According to the configurations described above, a gas treating apparatus and a gas treating method in which moisture is supplied in an efficient manner and amount when decomposing a VOC-containing gas to be treated using light having a main peak wavelength of 160 to 180 nm.

### BRIEF DESCRIPTION OF THE DRAWINGS

Fig. 1 is a schematic illustration of one embodiment of a gas treating apparatus.
Fig. 2 is a cross-sectional view seen from the direction of arrows A-A in Fig. 1.
Fig. 3 is a cross-sectional view seen from the direction of arrows B-B in Fig. 1.
Fig. 4 is a graph showing an emission spectrum of a xenon excimer lamp, overlapped with an oxygen (O₂) absorption spectrum and an ozone (O₃) absorption spectrum.
Fig. 5 is a graph showing a relationship between the distance from a light-emitting surface of the excimer lamp and the hydroxyl radical concentration.
Fig 6 is a schematic illustration of a gas treatment experiment system.
Fig. 7 is a graph showing a relationship between water vapor amount and VOC removal amount when a gas to be treated containing toluene is irradiated with 172 nm light in the first embodiment.
Fig. 8 is a graph showing a relationship between water vapor amount and VOC removal amount when a gas to be treated containing formaldehyde is irradiated with 172 nm light in the first embodiment.
Fig. 9 is a diagram illustrating a third embodiment of the gas treating apparatus.
Fig. 10 is a graph showing a relationship between water vapor amount and VOC removal amount in the third embodiment.
Fig. 11 is a diagram illustrating a fourth embodiment of the gas treating apparatus.

### EMBODYIMENTS FOR CARRYING OUT INVENTION

### < First embodiment>

### [Configuration of gas treating apparatus]

One embodiment of a gas treating apparatus according to the present invention will be described with reference to the drawings. It should be noted that the drawings disclosed herein merely show schematic illustrations. Namely, the dimensional ratios on the drawings do not necessarily reflect the actual dimensional ratios, and the dimensional ratios are not necessarily the same between the drawings.

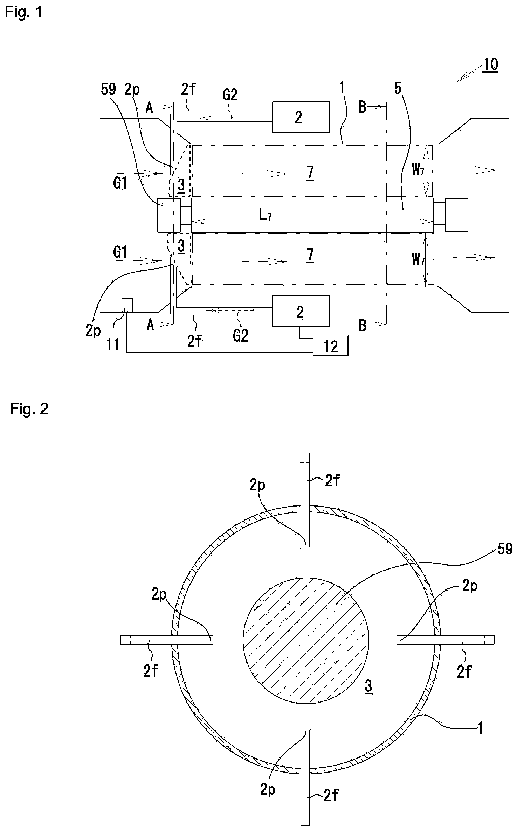

Fig. 1 is a schematic illustration of one embodiment of the gas treating apparatus. The gas treating apparatus 10 shown in Fig. 1 includes a housing 1 and a light source accommodated inside the housing 1. An excimer lamp 5 formed by a tubular body extending in one direction is illustrated as one example of the light source.

The gas treating apparatus 10 is an apparatus having a function of decomposing VOCs by irradiating a gas (hereinafter referred to as "gas to be treated") containing VOCs (Volatile Organic Compounds) with light of a predetermined wavelength (ultraviolet) from a light source (which corresponds to the excimer lamp 5 in Fig. 1). VOCs stand for volatile organic compounds in gaseous form at normal temperature and pressure, which are substances that cause indoor and outdoor air pollution. Typical substances that contain VOCs are toluene, formaldehyde, xylene, benzene, methanol, isopropyl alcohol, acetone, methyl isobutyl ketone, ethyl acetate, and so on. The gas to be treated flows generally in directions indicated with broken line arrows inside the housing 1.

The gas to be treated contains moisture and oxygen other than VOCs. As will be explained later why, moisture contained in the gas to be treated exists as vapor (gas), and the water vapor amount contained in the gas to be treated is 9 g/m³ or more. The gas to be treated is generated by mixing a first gas G1 containing VOCs and oxygen, and a second gas G2 containing moisture. In this embodiment, the second gas G2 is supplied from second gas supply units 2 through flow paths 2f into the flows of the first gas G1 passing through the housing 1, and mixed therewith in a gas mixing space 3 (inside the broken line frames in Fig. 1).

The first gas G1 is introduced from a first gas introducing unit (not shown in Fig. 1). The first gas introducing unit corresponds for example to an outlet port of a chamber space such as a draft chamber or the like. A blower (not shown in Fig. 1) is disposed downstream of the gas treating apparatus 10. By driving the blower, the first gas G1 is sucked and introduced from the first gas introducing unit, and the first gas G1 thus flows inside the housing 1.

The draft chamber and other spaces are often placed in an atmospheric environment. Therefore, generally, the first gas G1 contains not just VOCs but also oxygen in the atmosphere. In the case of decomposing or removing VOCs that were created in a special environment without the air or with little oxygen, oxygen may be added to VOCs intentionally to generate the first gas G1. The first gas G1 often contains some water vapor, too, because the atmosphere often contains water vapor.

The second gas G2 is air supplied in a state in which water vapor is contained. Fig. 2 is a cross-sectional view seen from the direction of arrows A-A in Fig. 1. The housing 1 is formed in a cylindrical shape. An end portion 59 of the excimer lamp 5 is located in the center of the housing 1. Four flow paths 2f for supplying the second gas G2 are equally spaced apart by the same angle such as to surround the housing 1. Preferably, the flow paths 2f are each connected to and branched from one second gas supply unit 2. The second gas G2 is ejected from a jet port 2p of each flow path 2f. The ejected second gas G2 is mixed with the first gas G1 in the gas mixing space 3 to produce a gas to be treated (G1 + G2). As will be explained later why, the gas to be treated contains 9 g/m³ or more of water vapor.

The gas to be treated is irradiated with light from the excimer lamp 5 in a gas treatment space 7 (inside the one-dot chain line frames in Fig. 1). The gas treatment space 7 has a size that accommodates a length L₇ in the tube axis direction on a light-emitting surface of the excimer lamp 5, and a clearance distance W₇ that is a length from the light-emitting surface of the excimer lamp 5 to an inner wall of the housing 1. As will be explained later why, the clearance distance W₇ is within 10 mm. That is to say, the gas to be treated is guided to a region within 10 mm of the light-emitting surface of the excimer lamp 5.

The excimer lamp 5 radiates light having a main peak wavelength of 160 to 180 nm. The light having a main peak wavelength of 160 to 180 nm is part of a wavelength range that is called VUV (Vacuum Ultra Violet). The light has a shorter wavelength than that of ultraviolet emitted in hitherto known low-pressure mercury-vapor lamps (e.g., light having a main peak wavelength of 185 nm). A typical example of light sources that generate light having a main peak wavelength of 160 to 180 nm is the excimer lamp hermetically filled with gases containing xenon (Xe), a typical peak wavelength of the excimer lamp generally being 172 nm. In the following description, an expression "172 nm light" is sometimes used instead of "light having a main peak wavelength of 160 to 180 nm". There is no particular difference in characteristics between "172 nm light" and "light having a main peak wavelength of 160 to 180 nm" in regard to the contents herein. Since any description relating to "172 nm light" hereinafter can also apply to "light having a main peak wavelength of 160 to 180 nm", the "172 nm light" mentioned hereinafter can be read as "light having a main peak wavelength of 160 to 180 nm".

The gas to be treated that has passed through the gas treatment space 7 is either sent to a gas collection tank as treated gas, or released to the atmosphere.

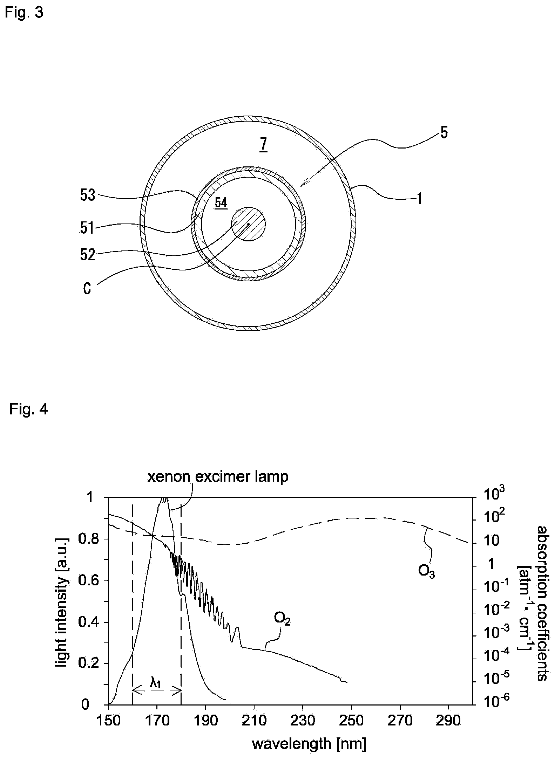

Fig. 3 is a cross-sectional view seen from the direction of arrows B-B in Fig. 1. The excimer lamp 5 is disposed in the center of the cylindrical housing 1 along the center axis C of the cylinder of the housing. The gas treatment space 7 is formed between the light-emitting surface of the excimer lamp 5 and the inner wall of the housing 1. One embodiment of the excimer lamp 5 is illustrated below.

In this embodiment, the excimer lamp 5 takes on a structure referred to as single tube type. Namely, the lamp has a tubular body 51 made of a dielectric material such as synthetic quartz glass, a first electrode 52 disposed inside the tubular body 51 with a space 54 from the tubular body 51, and a second electrode 53 disposed on an outer wall surface of the tubular body 51. The space 54 is filled with discharge gases containing xenon. The first electrode 52 contains the center axis C, and extends along a direction in which the center axis C extends. The center axis C of the first electrode 52 may coincide with the center axis of the cylindrical housing 1. The second electrode 53 is in a mesh-like or wire-like form, for example.

When a high-frequency alternating voltage of about 50 kHz to 5 MHz, for example, is applied across the first electrode 52 and the second electrode 53 via a power supply line which is not shown, from a lighting power source which is not shown, electrons of discharge gases filling the space 54 are excited to the excimer state, and excimers emit light when the atoms transition to the ground state. The excimer light transmits the tubular body 51, and through the gaps in the second electrodes 53, and irradiates the gas to be treated that is flowing through the gas treatment space 7.

The excimer lamp 5 may be a lamp having a structure known as "double tube structure," in which one tubular body is inside another. It may also be a lamp having a flat-shape tubular body with electrodes disposed on both ends. Alternatively, a plurality of excimer lamps 5 may be lined up in series along the flow path of the gas to be treated, or in parallel to the flow path of the gas to be treated.

As described above, in this embodiment, the first gas G1 containing VOCs and oxygen, and the second gas G2 containing water vapor, are mixed together to produce a gas to be treated that contains VOCs, oxygen, and 9 g/m³ or more of water vapor. The gas to be treated thus generated was guided to the region within 10 mm of the light-emitting surface of the excimer lamp 5 and irradiated with 172 nm light. This was done based on the findings obtained through the following verification and experiment.

### [Verification]

Water and oxygen are sources for producing hydroxyl radicals that actto decompose VOCs. The reason for this is shown by the following reaction formulas. In the following reaction formulas, O(¹D) represents a highly reactive oxygen atom, OH represents a hydroxyl radical, O(³P) represents a ground-state oxygen atom, and hv represents the absorbed energy of 172 nm light.

hv + H₂O → ·OH + H ··· (1)

hv + O₂ → O(¹D) + O(³P) ··· (2)

O(¹D) + H₂O → 2(·OH) ··· (3)

O(³P) + O₂ + M → O₃ + M ··· (4)

hv + O₃ + M → O(¹D) + O(³P) ··· (5)

The above formula (1) indicates how a hydroxyl radical is generated when the 172 nm light is absorbed in water. The reaction itself of formula (1) can occur with light having a main peak wavelength of 185 nm, for example. However, the reaction of formula (1) occurs far more easily with the 172 nm light than with the light of 185 nm and therefore the 172 nm light produces a large amount of hydroxyl radicals.

The above formula (2) indicates how O(¹D) is generated when the 172 nm light is absorbed in oxygen. Fig. 4 shows an emission spectrum of a xenon excimer lamp, overlapped with an oxygen (O₂) absorption spectrum and an ozone (O₃) absorption spectrum. In Fig. 4, the horizontal axis represents the wavelength, the left vertical axis represents the relative value of light intensity of the excimer lamp, and the right vertical axis represents the absorption coefficients of oxygen (O₂) and ozone (O₃). As shown in Fig. 4, the light emitted by the xenon excimer lamp includes a component in the wavelength range λ₁ of 160 to 180 nm. Since oxygen (O₂) absorbs a large amount of this wavelength range λ₁ light, the gas to be treated containing oxygen flowing near the excimer lamp absorbs most of the light of the wavelength range λ₁. Contrarily, oxygen (O₂) absorbs only a small amount of light having a wavelength longer than 180 nm (e.g., light of 185 nm). This means that the reaction of formula (2) hardly occurs with the light of wavelengths longer than 180 nm.

The O(¹D) generated by formula (2) then produces a hydroxyl radical by the reaction of the above formula (3). Thus the 172 nm light generates a large amount of hydroxyl radicals through reactions of formula (2) and formula (3).

The above formula (4) and formula (5) show how ozone (O₃) is produced from the O(³P) generated by formula (2) and oxygen, and how O(¹D) is produced by irradiating the generated ozone with the 172 nm light. Hydroxyl radicals are produced from the O(¹D) thus generated and water by formula (3). What is shown here is that O(³P) produced by formula (2) can generate hydroxyl radicals through production of ozone and O(¹D) if oxygen and water are present.

As shown above, the use of 172 nm light enables efficient production of a large amount of hydroxyl radicals from water and oxygen.

Next, the moisture contained in the gas to be treated is discussed. Moisture could be supplied, for example, in liquid form, such as water ejected from a showerhead, or sprayed in droplets, or could be supplied as water vapor, i.e., in gaseous form. To enhance the reaction of the above formula (1), it is desirable to increase the probability of contact between the 172 nm light and water molecules, and to enhance the reaction of the above formula (3), it is desirable to increase the probability of contact between O(¹D) and water molecules. To increase the probability of contact between light or O(¹D) and water molecules, it is suited to supply moisture in vapor (gaseous) form, which allows a larger region of contact for water molecules per unit space, i.e., which has a higher degree of dispersion of water molecules in unit space.

As discussed above, irradiation of the gas to be treated containing VOCs, oxygen and water vapor with the 172 nm light produces a large amount of hydroxyl radicals, whereby a high capability of decomposing and removing VOCs can be achieved.

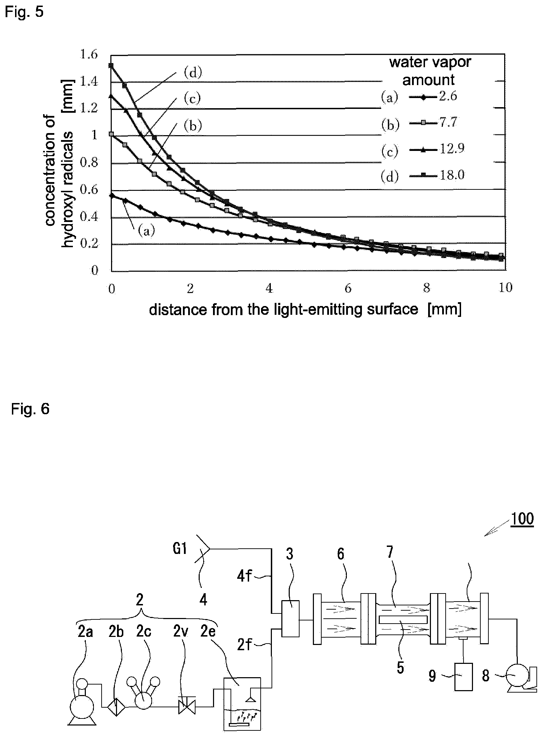

Fig. 5 is a result obtained by simulation showing a relationship between the distance from the light-emitting surface of the excimer lamp and the concentration of hydroxyl radicals generated by irradiation with 172 nm light. The graph shows the concentration of hydroxyl radicals generated by 172 nm light in air atmospheres respectively containing (a) 2.6 g/m³, (b) 7.7 g/m³, (c) 12.9 g/m³, and (d) 18.0 g/m³ of water vapor. As seen from Fig. 5, the higher the water vapor amount, the higher the concentration of hydroxyl radicals, in regions relatively in close distance from the light-emitting surface. This indicates that the higher the water vapor amount, i.e., the more the atmosphere contains oxygen and water molecules, the more easily the 172 nm light is absorbed by the oxygen and water molecules. Moreover, the closer it is to the light-emitting surface, the higher the concentration of hydroxyl radicals. It is also seen that, the more it is away from the light-emitting surface, the less the difference in hydroxyl radical concentration is attributed to the difference in water vapor amount. In particular, when the distance from the light-emitting surface exceeds 6 mm, the hydroxyl radical concentration reduces irrespective of the water vapor amount. It is assumed that no light reaches regions over 6 mm from the light-emitting surface because the 172 nm light is absorbed by oxygen molecules and the light energy disappears when the distance from the light-emitting surface exceeds 6 mm.

How long the clearance distance W₇ (length from the light-emitting surface of the excimer lamp 5 to an inner wall of the housing 1, see Fig. 1) in the gas treatment space 7 is a matter of importance. At first it was expected, from the results of Fig. 5, that VOCs passing through regions away from the light-emitting surface where the 172 nm light does not reach might travel through the gas treatment space 7 without being decomposed if the clearance distance W₇ was more than 6 mm.

However, extensive research revealed that, even if the clearance distance W₇ was over 6 mm, VOCs passing through regions away from the light-emitting surface could be relatively prevented from traveling through the gas treatment space 7 without being decomposed if the clearance distance W₇ was 15 mm or less. The reason for this is that the flow of the gas to be treated in the gas treatment space 7 is turbulent, constantly changing the distance from the light-emitting surface, rather than laminar, i.e., in layers parallel to the excimer lamp 5. Namely, if the gas treatment space 7 has a clearance distance W₇ of 15 mm or less, it is often the case that the gas to be treated present in regions of more than the clearance distance W₇ of 6 mm in the gas treatment space 7 enters the region within the clearance distance W₇ of 6 mm where the light reaches, somewhere in the gas treatment space 7, because of the turbulent flow. With a large clearance distance W₇, the flow rate of the gas to be treated passing through the gas treatment space 7 can be reduced, which consequently allows a sufficient time for the optical reaction of the gas to be treated.

It is particularly preferable to set the clearance distance W₇ within 10 mm. That way, VOCs traveling through a region away from the light-emitting surface can be prevented from passing through the gas treatment space 7 without being decomposed, and also, the flow rate of the gas to be treated passing through the gas treatment space 7 can be reduced so that there is enough time secured for the optical reaction of the gas to be treated.

### [Experiment]

In the embodiment described above, a second gas G2 containing 9 g/m³ or more of water vapor is supplied to a first gas G1 containing VOCs and oxygen. This is based on the findings obtained through the following experiment.

Fig 6 is a schematic illustration of a gas treatment experiment system. The experiment system 100 includes a second gas supply unit 2, a gas mixing space 3, a first gas introducing unit 4, an excimer lamp 5, a gas treatment space 7, a blower 8, and a VOC detector 9. Driving the blower 8 causes the first gas G1 (air containing VOCs) to be sucked from the first gas introducing unit 4 into the gas mixing space 3 through a flow path 4f.

The gas mixing space 3 is connected to the second gas supply unit 2 via a flow path 2f. The second gas supply unit 2 includes, for example, a compressor 2a, a dehumidifier filter 2b, a pressure adjuster 2c, a flow amount adjustment valve 2v, and a humidifier 2e. The compressor 2a takes in and compresses the air etc., in the atmosphere. The dehumidifier filter 2b removes moisture contained in the compressed gas. The dehumidifier filter 2b may be a membrane filter, or may be configured as a space filled with an adsorbent such as zeolite, silica gel, and so on. Alternatively, a mechanism that cools the gas for forced condensation may be disposed instead of the dehumidifier filter 2b to remove the moisture contained in the air.

After the pressure and flow amount have been adjusted by the pressure adjuster 2c and flow amount adjustment valve 2v, a desired water vapor amount is added to the dehumidified gas by the humidifier 2e. The water vapor amount contained in the second gas G2 is controlled this way. In the case where the compressor 2a takes in and compresses the atmosphere, the gas supplied from the second gas supply unit 2 would also contain oxygen in the atmosphere. The second gas G2 thus containing water vapor of a required amount in the air is sent to the gas mixing space 3 through the flow path 2f.

The first gas G1 containing VOCs and air (oxygen) and the second gas G2 containing water vapor are mixed in the gas mixing space 3 to produce a gas to be treated. After a flow regulator 6 has removed fluctuations in the flow rate, the gas to be treated is sent to the gas treatment space 7. The gas mixing space 3 in the experiment system 100 is distanced from the gas treatment space 7 and includes the flow regulator 6 between these spaces, as opposed to the gas mixing space 3 located immediately before the gas treatment space 7 in the gas treating apparatus 10. This difference does not cause a difference in the amount of VOCs that are removed.

In the experimental condition, the gas treatment space 7 was configured by a hollow housing of SUS304. The excimer lamp 5 was disposed inside the housing as illustrated in Fig. 1. The excimer lamp 5 was an excimer lamp filled with discharge gases containing xenon (172 nm light), with a length L₇ of the light-emitting part in the tube axis direction of 130 mm. The tubular body 51 is 16 mm in diameter. The clearance distance W₇ was 10 mm. The inverter input power to the excimer lamp was 31.7 W. The experiment was conducted using air containing 10 ppm toluene as the first gas G1 to be supplied, while the concentration of the water vapor contained in the gas to be treated was varied. The flow rate of the first gas G1 was 20 L per minute.

After treating the gas to be treated in the gas treatment space 7, the VOC concentration of the treated gas that has passed through the gas treatment space 7 was detected with the VOC detector 9. As the VOC detector 9 for detecting the VOC concentration, Tiger, a VOC concentration meter by RIKEN KEIKI CO., LTD., was used. The VOC concentration is detected with the excimer lamp 5 emitting light, and the VOC concentration is detected also with the excimer lamp 5 not emitting light. The difference in these VOC concentrations indicates the amount of VOCs that were removed by means of the excimer lamp 5. The amount of removed VOCs may be computed from a difference in readings of the VOC detector 9 and another VOC detector disposed upstream of the gas treatment space 7.

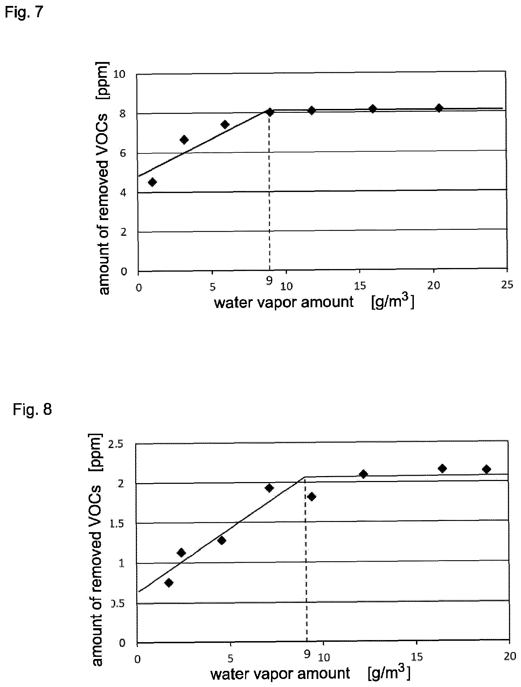

Fig. 7 is a graph showing the relationship between the water vapor amount and the amount of removed VOCs measured under the experimental conditions described above, where the gas to be treated containing toluene was irradiated with the 172 nm light in the gas treatment space 7, while the water vapor amount contained in the gas to be treated was varied.

From the results shown in Fig. 5 that indicate a rise in the concentration of hydroxyl radicals having a high ability to decompose VOCs proportional to the increase in water vapor amount, it was expected at first that the amount of removed VOCs would rise with the increase in water vapor amount. However, the results obtained through the experiment revealed a surprising fact that, as shown in Fig. 7, while the VOC removal amount rose with an increase in the water vapor amount when the water vapor amount was lower than 9 g/m³, the VOC removal amount did not rise with an increase in the water vapor amount when the water vapor amount was 9 g/m³ or more. The present inventor assumes that this is because of more frequent occurrence of self-combination of increased hydroxyl radicals when there is 9 g/m³ or more of water vapor so that the increased hydroxyl radicals are not made available for the removal of VOCs. The results of this experiment show that it is beneficial for efficient removal of VOCs if the gas to be treated contains 9 g/m³ or more of water vapor.

Next, an experiment was conducted using air containing 10 ppm formaldehyde, instead of the air containing 10 ppm toluene, as the first gas G1 to be supplied, while the concentration of the water vapor contained in the gas to be treated was varied. The flow rate of the first gas G1 was 20 L per minute. Other experimental conditions were the same as those specified above. Fig. 8 shows the results of the experiment. Fig. 8 is a graph showing the relationship between the water vapor amount and the amount of removed VOCs that was measured when the gas to be treated containing formaldehyde was irradiated with the 172 nm light in the gas treatment space 7, while the water vapor amount contained in the gas to be treated was varied. Similarly to Fig. 7 (which shows the results of the experiment in which air containing 10 ppm toluene was used as the first gas G1), the results obtained through the experiment revealed that, while the VOC removal amount rose with an increase in the water vapor amount when the water vapor amount was lower than 9 g/m³, the VOC removal amount did not rise with an increase in the water vapor amount when the water vapor amount was 9 g/m³ or more. This indicates that the threshold value of 9 g/m³ does not change depending on the type of VOC. This is considered to be because the behaviors of hydroxyl radicals to the 172 nm light are hardly affected by the type of VOC.

### <Second embodiment>

The second embodiment is similar in configuration to the first embodiment except for the following points. Therefore, description of the common points will be omitted. The same applies, too, from the third embodiment onward.

The gas treating apparatus 10 includes a water vapor amount detector 11 that detects a water vapor amount contained in a first gas G1 that contains VOCs and oxygen, and a controller 12 that adjusts the water vapor amount supplied from the second gas supply unit 2 based on a detection result of the water vapor amount detector 11. Referring to the gas treating apparatus 10 shown in Fig. 1, the water vapor amount detector 11 is provided to one side of the gas treating apparatus 10 where the first gas G1 flows in, and electrically connected to the controller 12. The controller 12 is electrically connected to the second gas supply unit 2. When the water vapor amount contained in the first gas G1 detected by the water vapor amount detector 11 is less than 9 g/m³, that controller 12 may control the second gas supply unit 2 to supplement water vapor in the amount lacking of 9 g/m³, for efficient removal of VOCs. For the water vapor amount detector 11, an electronic hygrometer, for example, is used.

In the case where the first gas G1 contains 9 g/m³ or more of water vapor, there is no need to supply the second gas G2 containing water vapor from the second gas supply unit 2. Namely, in this case, the gas treatment is performed by irradiating only the first gas G1.

When the water vapor amount contained in the gas to be treated exceeds the saturation moisture content of the gas to be treated so that the gas to be treated starts to condense inside the gas treatment space 7, less hydroxyl radicals are generated. Moreover, if the gas to be treated that flows in contact with the second electrode 53 of the excimer lamp 5 (see Fig. 3) condenses on the second electrode 53, the excimer lamp 5 could be damaged by concentrated discharge that may occur due to a local change in resistance between the electrodes. Therefore, it is preferable to keep the water vapor amount in the gas to be treated lower than the saturation moisture content to avoid condensation of the gas to be treated. Given that the saturation moisture content at 23°C close to room temperature is 20.6 g/m³, the water vapor amount contained in the gas to be treated should preferably be 20 g/m³ or less. More preferably, the water vapor amount contained in the gas to be treated should be 15 g/m³ or less.

Needless to say, the gas to be treated with a water vapor amount of 20 g/m³ or less, or 15 g/m³ or less, may still condense if the temperature is low. When the temperature of the gas to be treated is low, it is preferable to dispose the flow path of the gas to be treated closer to a heat source such as the excimer lamp, or, to provide some simple heating mechanism, to raise the temperature of the gas to be treated to a level where condensation can be prevented (e.g., 23°C).

In view of the risk of condensation, the gas to be treated should preferably have a relative humidity of 80% or less. In the case where the water vapor amount contained in the first gas G1 is more than 20 g/m³ (or 15 g/m³), or where the first gas G1 contains droplets such as mist, the moisture contained in the VOC gas may be removed by providing a dehumidifier filter or a space filled with an adsorbent such as zeolite, silica gel, etc., midway in the flow path 4f, or, by cooling the gas to force condensation.

### <Third embodiment>

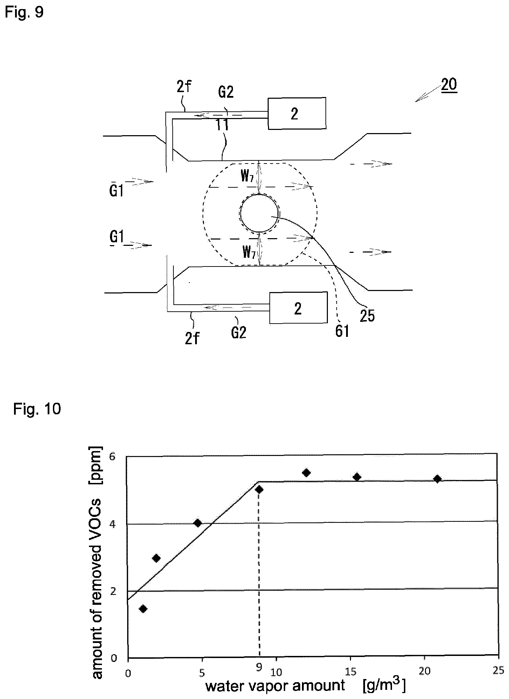

Fig. 9 illustrates a third embodiment of the gas treating apparatus. The excimer lamp 25 of the gas treating apparatus 20 is a cylindrical member that is 9 mm in diameter and 14 mm in length in the longitudinal direction. The excimer lamp 25 is disposed such that its longitudinal direction is perpendicular to the page. The clearance distance W₇ is 10 mm. The inverter input power to the excimer lamp 25 is 3 W.

Fig. 10 is a graph showing the relationship between the water vapor amount and the amount of removed VOCs that was measured under the experimental conditions described above, where the gas to be treated was irradiated with the 172 nm light in the gas treatment space 7, while the water vapor amount contained in the gas to be treated was varied. The flow rate of the gas to be treated is 1 L per minute. A gas containing 10 ppm toluene is used as the gas to be treated.

As shown in Fig. 10, the results indicated that, while the VOC removal amount rose with an increase in the water vapor amount when the water vapor amount was lower than 9 g/m³, the VOC removal amount did not rise with an increase in the water vapor amount when the water vapor amount was 9 g/m³ or more, similarly to Fig. 7. Fig. 7 and Fig. 10 indicate that, even if the position, size, and output etc., of the excimer lamp were different, the water vapor amount of 9 g/m³ or more of the gas to be treated necessary for efficient removal of VOCs would not change. To increase the overall VOC removal amount, it is effective to change the type, dimensions, and output of the excimer lamp, or to increase the number of excimer lamps.

### <Fourth embodiment>

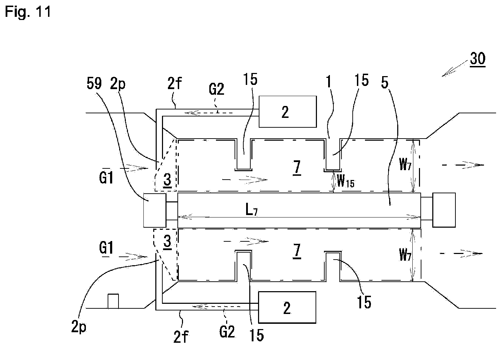

Fig. 11 illustrates a fourth embodiment of the gas treating apparatus. The gas treating apparatus 30 according to this embodiment includes protruded parts 15 in the housing 1. The distance W₁₅ from the light-emitting surface of the excimer lamp 5 and the protruded parts 15 is set such as to allow the light to likely reach (e.g., 6 mm or less). Since the entire gas to be treated passes through between the light-emitting surface of the excimer lamp 5 and the protruded parts 15, the entire amount of the gas to be treated can reliably be irradiated with light. In the case where such protruded parts 15 are provided, the clearance distance W₇ from the light-emitting surface of the excimer lamp 5 to the housing 1 in other parts than the protruded parts 15 may be set such as not to allow the light to reach the gas to be treated at least partly (e.g., more than 15 mm).

The present invention is not limited to the embodiments described above in any way. Various improvements and modifications may be made without departing from the scope of the subject matter of the present invention.

### DESCRIPTION OF REFERENCE SIGNS

- 1:: Housing
- 2: Second gas supply unit
- 2a: Compressor
- 2b: Dehumidifier filter
- 2c: Pressure adjuster
- 2e: Humidifier
- 2f: Flow path
- 2p: Jet port
- 2v: Flow amount adjustment valve
- 3: Gas mixing space
- 4: First gas introducing unit
- 4f: Flow path
- 5, 25: Excimer lamp
- 6: Flow regulator
- 7: Gas treatment space
- 8: Blower
- 9: VOC detector
- 10, 20, 30: Gas treating apparatus
- 11: Water vapor amount detector
- 12: Controller
- 15: Protruded part
- 51: Tubular body
- 52: First electrode
- 53: Second electrode
- 54: Space (between tubular body 51 and first electrode 52)
- 59: End portion
- 100: Experiment system
- G1: First gas
- G2: Second gas

## Claims

1. A gas treating apparatus comprising:
a gas mixing space where a first gas containing a VOC and oxygen and a second gas containing water vapor are mixed to generate a gas to be treated that contains a VOC, oxygen, and 9 g/m³ or more of water vapor;
a light source radiating light having a main peak wavelength of 160 to 180 nm; and
a gas treatment space where the gas to be treated is guided to a region within 10 mm of a light-emitting surface of the light source and the gas to be treated guided to the region is irradiated with the light.

2. The gas treating apparatus according to claim 1, further comprising:
a second gas supply unit that supplies the second gas to the gas mixing space;
a water vapor amount detector that detects a water vapor amount contained in the first gas; and
a controller that adjusts a water vapor amount contained in the second gas supplied from the second gas supply unit based on a detection result of the water vapor amount detector.

3. The gas treating apparatus according to claim 1 or 2, wherein the gas to be treated contains 20 g/m³ or less of water vapor.

4. The gas treating apparatus according to any one of claims 1 to 3, wherein the VOC includes toluene or formaldehyde.

5. A gas treating method comprising:
an introducing step (a) of causing a gas to be treated containing a VOC, oxygen, and 9 g/m³ or more of water vapor to flow to a region within 10 mm of a light-emitting surface of a light source radiating light having a main peak wavelength of 160 to 180 nm; and
an irradiation step (b) of irradiating the gas to be treated that has flowed to the region with the light.

6. The gas treating method according to claim 5, further comprising:
a gas to be treated generation step (c) in which a first gas containing a VOC and oxygen and a second gas containing water vapor are mixed together to produce the gas to be treated, before the irradiation step (b).

7. The gas treating method according to claim 6, wherein the gas to be treated generation step (c) includes
a detection step of detecting a water vapor amount contained in the first gas; and
a step of adjusting a water vapor amount in which a water vapor amount contained in the second gas is adjusted based on the water vapor amount detected in the detection step.

8. The gas treating method according to any one of claims 5 to 7, wherein the gas to be treated contains 20 g/m³ or less of water vapor.

9. The gas treating method according to any one of claims 5 to 8, wherein the VOC includes toluene or formaldehyde.
